# EUROPEAN PATENT APPLICATION

(11) **EP 1 895 004 A1**
(43) Date of publication of application: **05.03.2008**
(21) Application number: 06018330.8
(22) Date of filing: 01.09.2006
(51) Int. Cl.: C12N 15/12, C12N 15/63, C07K 14/47, C07K 16/18, C12N 5/10, C12Q 1/68, G01N 33/53, C12P 21/02

(54) **Human hippocalcin gene variant associated with cancers**

(71) Applicant: Visgeneer, Inc., Hsinchu City 300 (TW)
(72) Inventor: Dai, Ken-Shwo, Hsinchu City 300 (TW)
(74) Representative: Epping - Hermann - Fischer

(57) **Abstract**

The invention relates to the nucleic acid and polypeptide sequences of one novel human Hippocalcin- related gene variant (Hippocalcin-V).

The invention also provides a process for producing the polypeptides of the variant.

The invention further provides a use of the nucleic acid and polypeptide sequences of the gene variant to diagnose human large cell lung carcinoma, small cell lung carcinoma, colon adenocarcinoma and promonocytic leukemia.

## Description

### FIELD OF THE INVENTION

The invention relates to the nucleic acid and polypeptide sequences of a novel human Hippocalcin-related gene variant, preparation process thereof, and uses of the same in diagnosing cancers, in particular, large cell lung carcinoma, small cell lung carcinoma, colon adenocarcinoma and promonocytic leukemia.

### BACKGROUND OF THE INVENTION

Cancer is one of the major causes of death in the world. According to the report from the WHO Fact Sheet-Cancer (2003), cancer accounts for 7.1 million (12.6% of the global total) deaths annually and new cancer cases are expected to increase from 10 million people in 2000 to 15 million people by 2020. In recent years, progress has been made toward understanding the molecular and cellular biology of cancers. Many important contributions have been made by the identification of several key genetic factors associated with cancers. However, the treatments of cancers still mainly depend on surgery, chemotherapy and radiotherapy because the molecular mechanisms underlying the pathogenesis of cancers remain largely unclear.

It is interesting to note that a "remote effect" shown as paraneoplastic syndromes has been reported between neurological disorders and systemic cancers. Recoverin, a member of the neuronal calcium sensor family --- a group of neuronal expressed proteins containing calcium binding domains of the E-F hand structure (Burgoyne and Weiss, (2001) Biochem J. 353:1-12), has been reported to play an important role in the pathogenesis of the tumor cells (Polans et al., (1995) Proc Natl Acad Sci U S A. 92:9176-80; Maeda et al., (2000) Cancer Res. 60:1914-20; Maeda et al., (2001) Investigative Ophthalmology and Visual Science 42:705-712; Ohguro et al., (2004) Tohoku J Exp Med. 202:213-9). Furthermore, the involvement of neuronal calcium sensor protein in caspase-dependent apoptotic pathways has been reported to associate with the syndromes (Maeda et al., (2001) Investigative Ophthalmology and Visual Science 42:705-712). Therefore, future strategies for the prevention and treatment of cancers may be focused on the elucidation of the neuronal calcium sensor proteins that are involved in the caspase-dependent apoptotic pathways. Hippocalcin, a member of the neuronal calcium sensor protein family, has been shown to be involved in the cell apoptosis (Mercer et al. (2000) EMBO J. 19:3597-607) suggesting that Hippocalcin may play a role in the pathogenesis of cancers. Previous studies have shown that many gene variants have been found to be associated with diseases. Therefore, the discovery of gene variants for Hippocalcin may be an important goal for the development of diagnostic markers of cancers.

### SUMMARY OF THE INVENTION

The present invention provides one Hippocalcin-related gene variant present in human large cell lung carcinoma, small cell lung carcinoma, colon adenocarcinoma and promonocytic leukemia. The nucleotide sequence of the gene variant and polypeptide sequence encoded thereby can be used to diagnosis any diseases associated with this gene variant or cancers, in particular, large cell lung carcinoma, small cell lung carcinoma, colon adenocarcinoma and promonocytic leukemia.

The present invention further provides an expression vector and host cell for expressing the variant.

The invention further provides a method for producing the variant.

The invention also provides an antibody that specifically binds to the variant. For example, by selecting a sequence unique to one of the variants, e.g., a peptide fragment that spans on the deletion junctions, an antibody may be generated that binds to one of the variants and not to Hippocalcin. Preferably, such peptide antigens are at least 17 amino acid long, but in some cases smaller peptides such as 10mers, 12mers, or 15mers may suffice. Longer peptides, such as 20mers, 50mers, hundred-mers (and those therebetween) and even up to full length proteins may be used as well.

The invention also provides methods for detecting the presence of the variant in a mammal.

### BRIEF DESCRIPTION OF THE DRAWINGS

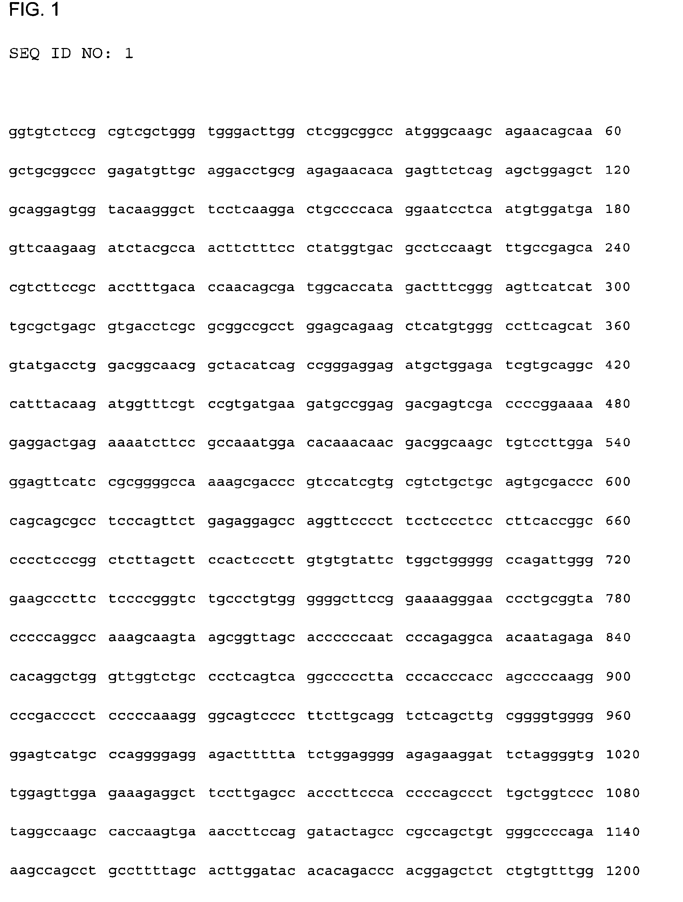

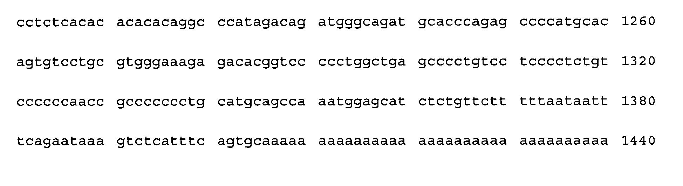

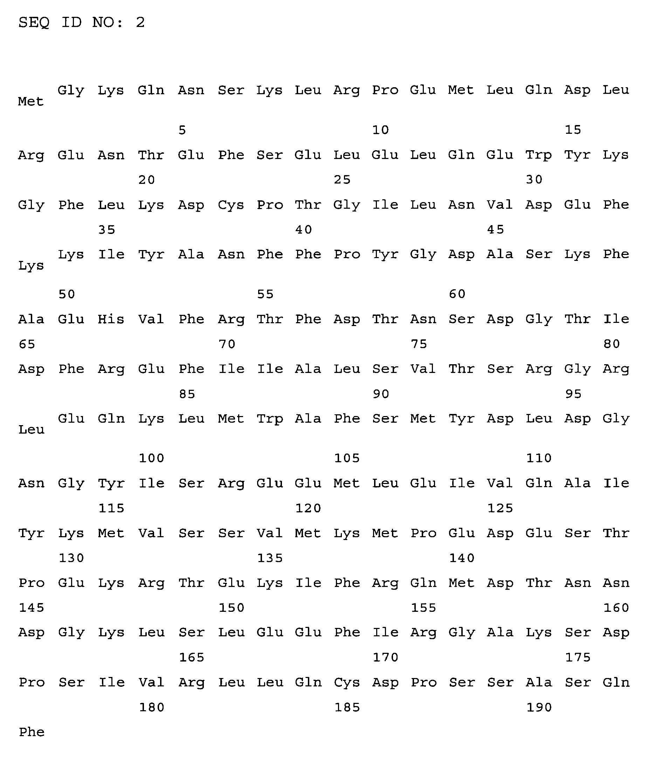

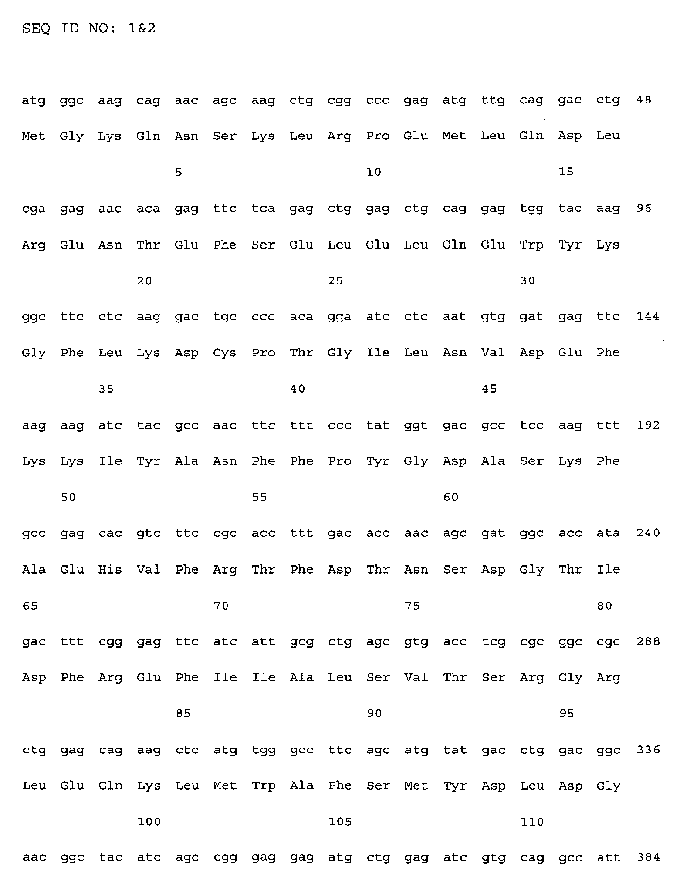

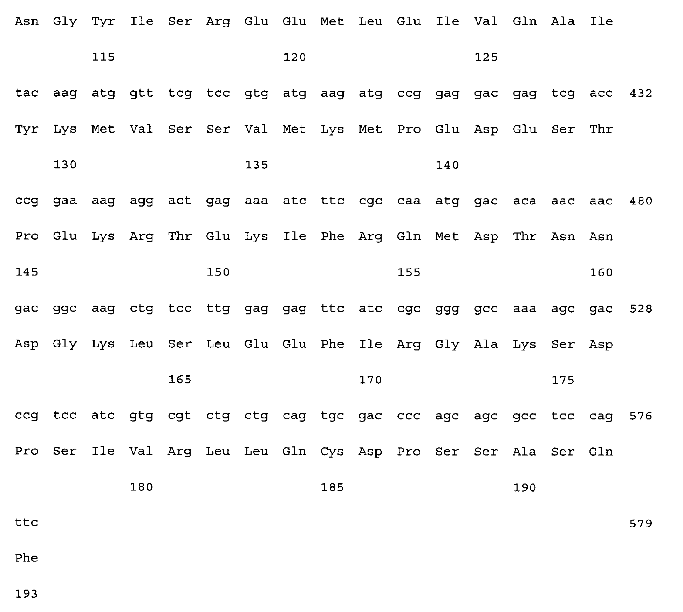

FIG. 1 shows the nucleic acid sequence (SEQ ID NO:1), amino acid sequence (SEQ ID NO:2) and nucleic acid with corresponding amino acid sequence (SEQ ID NO:1 &2) of Hippocalcin.

FIG. 2 shows the nucleic acid sequence (SEQ ID NO:3), amino acid sequence (SEQ ID NO:4) and nucleic acid with corresponding amino acid sequence (SEQ ID NO:3&4) of Hippocalcin-V.

FIG. 3 shows the nucleotide sequence alignment between the human Hippocalcin gene and its related gene variant (Hippocalcin-V).

FIG. 4 shows the amino acid sequence alignment between the human Hippocalcin protein and its related gene variant (Hippocalcin-V).

FIG. 5 shows the expression pattern of Hippocalcin-V in human cell lines.

### DETAILED DESCRIPTION OF THE INVENTION

All technical and scientific terms used herein, unless otherwise defined, have the same meanings as commonly understood by persons skilled in the art..

The term "antibody" used herein denotes intact molecules (a polypeptide or group of polypeptides) as well as fragments thereof, such as Fab, R(ab')₂, and Fv fragments, which are capable of binding the epitopes. Antibodies are produced by specialized B cells after stimulation by an antigen. Structurally, antibody consists of four subunits including two heavy chains and two light chains. The internal surface shape and charge distribution of the antibody binding domain is complementary to the features of an antigen. Thus, antibody can specifically act against the antigen in an immune response.

The term "base pair (bp)" used herein denotes nucleotides composed of a purine on one strand of DNA which can be hydrogen bonded to a pyrimidine on the other strand. Thymine (or uracil) and adenine residues are linked by two hydrogen bonds. Cytosine and guanine residues are linked by three hydrogen bonds.

The term "Basic Local Alignment Search Tool (BLAST; Altschul et al., (1997) Nucleic Acids Res. 25: 3389-3402)" used herein denotes programs for evaluation of homologies between a query sequence (amino or nucleic acid) and a test sequence. Specific BLAST programs are described as follows:

(1) BLASTN compares a nucleotide query sequence against a nucleotide sequence database;

(2) BLASTP compares an amino acid query sequence against a protein sequence database;

(3) BLASTX compares the six-frame conceptual translation products of a query nucleotide sequence against a protein sequence database;

(4) TBLASTN compares a query protein sequence against a nucleotide sequence database translated in all six reading frames; and

(5) TBLASTX compares the six-frame translations of a nucleotide query sequence against the six-frame translations of a nucleotide sequence database.

The term "cDNA" used herein denotes nucleic acids that synthesized from a mRNA template using reverse transcriptase.

The term "cDNA library" used herein denotes a library composed of complementary DNAs which are reverse-transcribed from mRNAs.

The term "complement" used herein denotes a polynucleotide sequence capable of forming base pairing with another polynucleotide sequence. For example, the sequence 5'-ATGGACTTACT-3' binds to the complementary sequence 5'- AGTAAGTCCAT-3'.

The term "deletion" used herein denotes a removal of a portion of one or more amino acid residues/nucleotides from a gene.

The term "expressed sequence tags (ESTs)" used herein denotes short (200 to 500 base pairs) nucleotide sequence that derives from either 5' or 3' end of a cDNA.

The term "expression vector" used herein denotes nucleic acid constructs which contain a cloning site for introducing the DNA into vector, one or more selectable markers for selecting vectors containing the DNA, an origin of replication for replicating the vector whenever the host cell divides, a terminator sequence, a polyadenylation signal, and a suitable control sequence which can effectively express the DNA in a suitable host. The suitable control sequence may include promoter, enhancer and other regulatory sequences necessary for directing polymerases to transcribe the DNA.

The term "host cell" used herein denotes a cell which is used to receive, maintain, and allow the reproduction of an expression vector comprising DNA. Host cells are transformed or transfected with suitable vectors constructed using recombinant DNA methods. The recombinant DNA introduced with the vector is replicated whenever the cell divides.

The term "insertion" or "addition" used herein denotes the addition of a portion of one or more amino acid residues/nucleotides to a gene.

The term "in silico" used herein denotes a process of using computational methods (e.g., BLAST) to analyze DNA sequences.

The term "polymerase chain reaction (PCR)" used herein denotes a method which increases the copy number of a nucleic acid sequence using a DNA polymerase and a set of primers (about 20bp oligonucleotides complementary to each strand of DNA) under suitable conditions (successive rounds of primer annealing, strand elongation, and dissociation).

The term "polynucleotide" or "nucleic acid sequence"used herein denotes a sequence of nucleotide (guanine, cytosine, thymine or adenine) in a specific order that can be a natural or synthesized fragment of DNA or RNA. It may be single-stranded or double-stranded.

The term "protein" or "polypeptide" used herein denotes a sequence of amino acids in a specific order that can be encoded by a gene or by a recombinant DNA. It can also be chemically synthesized.

The term "RNA interference (RNAi)" used herein denotes an introduction of homologous double stranded RNA (dsRNA) into a cell to specifically inhibit the expression of a gene.

The term "reverse transcriptase-polymerase chain reaction (RT-PCR)" used herein denotes a process which transcribes mRNA to complementary DNA strand using reverse transcriptase followed by polymerase chain reaction to amplify the specific fragment of DNA sequences.

The term "transformation" used herein denotes a process describing the uptake, incorporation, and expression of exogenous DNA by prokaryotic host cells.

The term "transfection" used herein denotes a process describing the uptake, incorporation, and expression of exogenous DNA by eukaryotic host cells.

The term "variant" used herein denotes a fragment of sequence (nucleotide or amino acid) inserted or deleted by one or more nucleotides/amino acids.

According to the present invention, the polypeptides of one novel human Hippocalcin-related gene variant and the nucleic acid sequences encoding the same are provided.

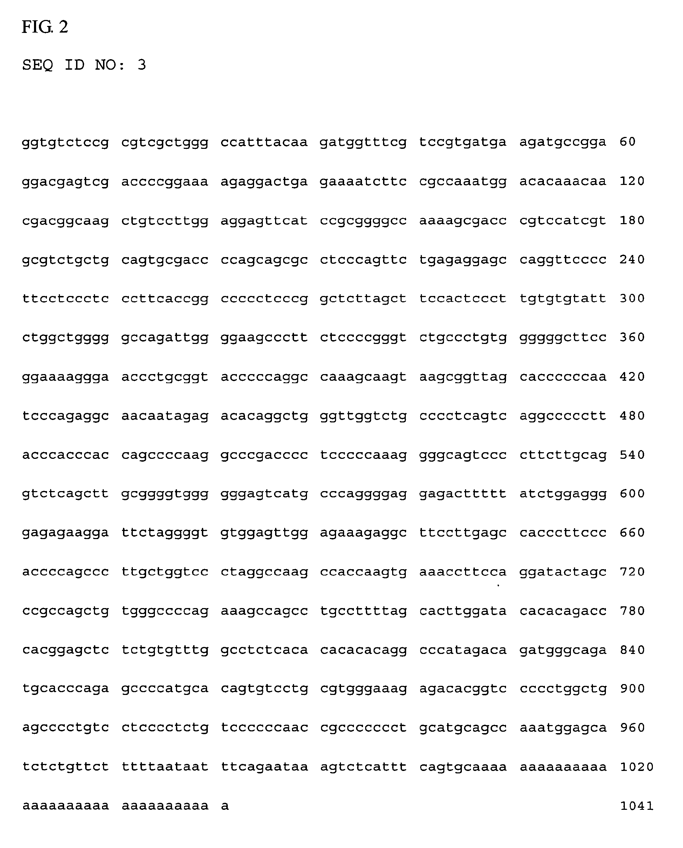

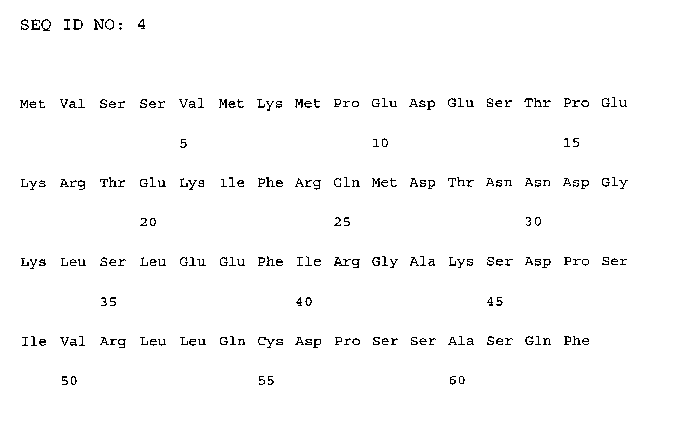

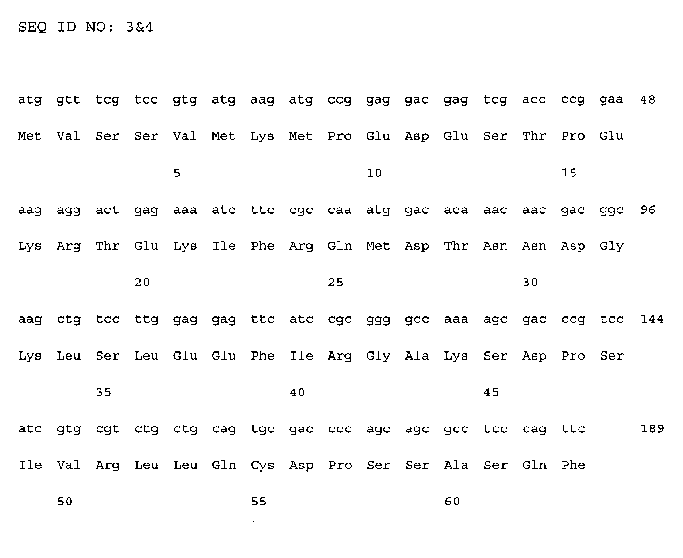

According to the present invention, human Hippocalcin cDNA sequence was used to query the human EST databases using BLAST program to search for Hippocalcin-related gene variants. One human cDNA partial sequences (i.e., EST) similar to Hippocalcin was identified from ESTs deposited in a cDNA database constructed using a H209 (Small cell lung carcinoma) cell line. The cDNA clone, named Hippocalcin-V (Hippocalcin variant), was then isolated from the H209 cDNA library and sequenced. FIG. 2 shows the nucleic acid sequences (SEQ ID NOs:3) of Hippocalcin-V and its corresponding amino acid sequences (SEQ ID NOs:4) encoded thereby.

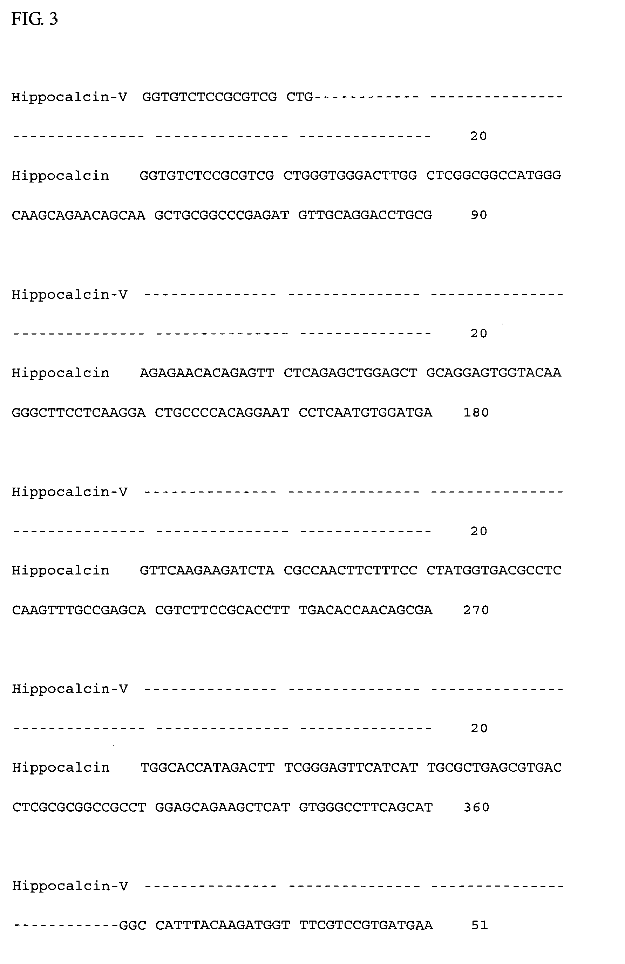

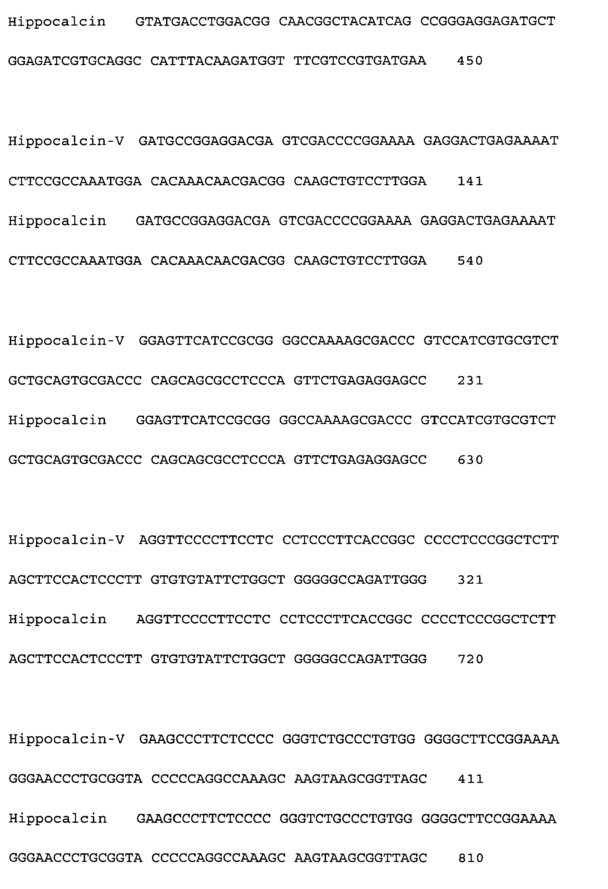

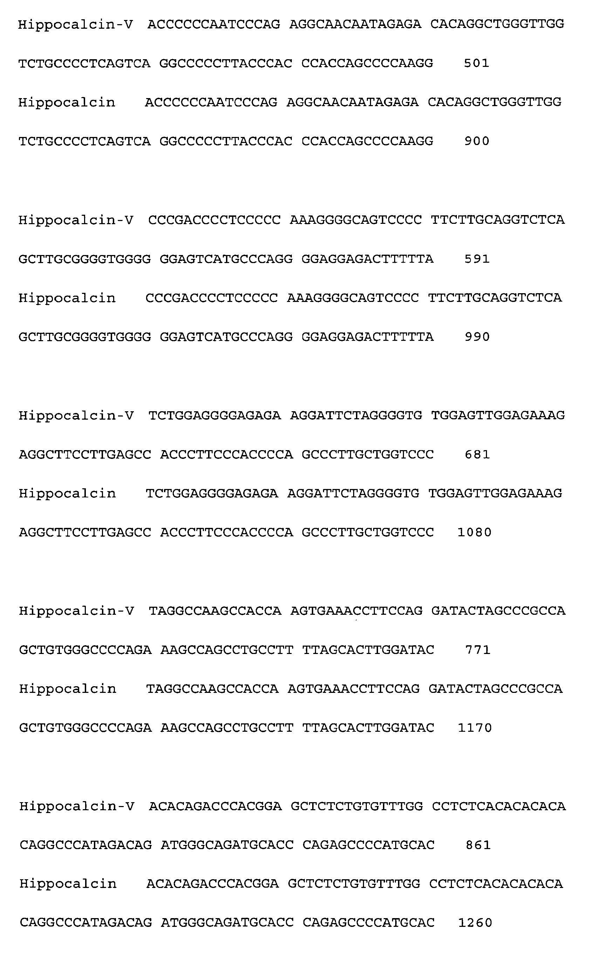

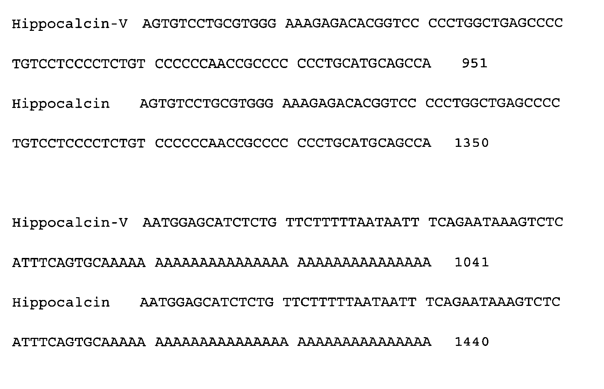

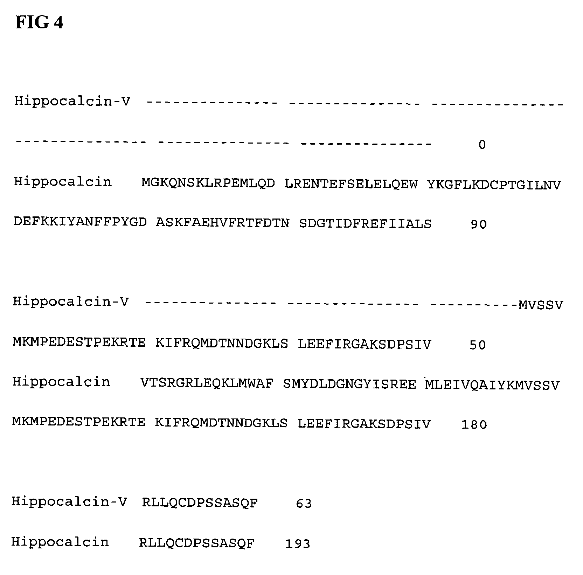

The full-length of the Hippocalcin-V cDNA is a 1041bp clone containing a 189bp open reading frame (ORF) extending from nucleotides 32 to 220, which corresponds to an encoded protein of 63 amino acid residues with a predicted molecular mass of 7.1 kDa. The initiation ATG sequence of Hippocalcin-V is located at nucleotide nucleotides 32-34. To determine the variation in sequence of Hippocalcin-V cDNA clone, an alignment of Hippocalcin nucleotide/amino acid sequence with Hippocalcin-V was performed (FIGs. 3 and 4). The results indicate that one major genetic deletion was found in the aligned nucleotide sequences, indicating that Hippocalcin-V has a 399bp deletion in the sequence of Hippocalcin from nucleotides 19-417. Thus, the Hippocalcin-V protein has a 133 amino acid N-terminal deletion comparing to the Hippocalcin protein. Scanning both Hippocalcin and Hippocalcin-V amino acid sequences against protein profile databases that include PROSITE (Motif Scan) indicated that Hippocalcin protein contains three EF-hand calcium-binding domain sites (60-95aa, 96-131aa, and 144-179aa). On the other hand, Hippocalcin-V protein contains only one EF-hand calcium-binding domain sites (14-49aa).

In the present invention, a search of ESTs deposited in dbEST at NCBI was performed to determine the presence of Hippocalcin-V *in silico.* The result of *in silico* analysis showed that two ESTs (GenBank accession number BP311792 and BI550996) were found to confirm the absence of 399bp region on Hippocalcin-V nucleotide sequence. Therefore, any Hippocalcin nucleotide fragment comprising the immediately upstream and downstream sequences of the deleted 399bp region is the gene target which may be used as probes to determine the presence of Hippocalcin-V under high stringency conditions. An alternative approach is that any set of primers for amplifying the fragment containing the immediately upstream and downsream sequences of the deleted 399bp region (nucleotide 18-19 of Hippocalcin-V) may be used to determine the presence of the variant.

According to the present invention, the polypeptides of the human Hippocalcin-V and its fragments thereof may be produced via genetic engineering techniques. In this case, they are produced by appropriate host cells which have been transformed by DNAs that code for the polypeptides or fragments thereof. The nucleotide sequence encoding the polypeptide of the human Hippocalcin-V or their fragments thereof is inserted into an appropriate expression vector, i.e., a vector which contains the necessary elements for the transcription and translation of the inserted coding sequence in a suitable host. The nucleic acid sequence is inserted into the vector where the sequence will be expressed under appropriate conditions (e.g., in proper orientation and correct reading frame and with appropriate expression sequences, including an RNA polymerase binding sequence and a ribosomal binding sequence).

Any method that is known to those skilled in the art may be used to construct expression vectors containing sequences encoding the polypeptides of the human Hippocalcin-V and appropriate transcriptional/translational control elements. These methods may include *in vitro* recombinant DNA and synthetic techniques, and *in vivo* genetic recombinants.

A variety of expression vector/host systems may be utilized to express the polypeptide-coding sequence. These include, but are not limited to, microorganisms such as bacteria transformed with recombinant bacteriophage, plasmid, or cosmid DNA expression vector; yeast transformed with yeast expression vector; insect cell systems infected with virus (e.g., baculovirus); plant cell system transformed with viral expression vector (e.g., cauliflower mosaic virus, CaMV, or tobacco mosaic virus, TMV); or animal cell system infected with virus (e.g., vaccina virus, adenovirus, etc.). Preferably, the host cell is a bacterium, and most preferably, the bacterium is *E*. *coli.*

Alternatively, the Polypeptides of the human Hippocalcin-V or fragments thereof may be synthesized using chemical methods. For example, peptide synthesis can be performed using various solid-phase techniques. Automated synthesis may be achieved using the ABI 431A peptide synthesizer (Perkin-Elmer).

According to the present invention, the fragments of the polypeptides and nucleic acid sequences of the human Hippocalcin-V are used as immunogens, primers or probes, respectively. Preferable, the purified fragments of the human Hippocalcin-V are used. The fragments may be produced by enzyme digestion, chemical cleavage of isolated or purified polypeptide or nucleic acid sequences, or chemical synthesis. The fragments then may be isolated or purified. Such isolated or purified fragments of the polypeptides and nucleic acid sequences can be used as immunogens, primers or probes, respectively.

The present invention further provides the antibodies which specifically bind one or more out-surface epitopes of the polypeptides of the human Hippocalcin-V.

According to the present invention, immunization of mammals such as humans, rabbits, rats, mice, sheep, goats, cows, or horses with immunogens described herin is performed using procedures well known to those skilled in the art, for the purpose of obtaining antisera containing polyclonal antibodies or hybridoma lines secreting monoclonal antibodies.

Monoclonal antibodies can be prepared by standard techniques, given the teachings contained herein. Such techniques are disclosed, for example, in U.S. patent number 4,271,145 and U.S. patent number 4,196,265. Briefly, an animal is immunized with the immunogen. Hybridomas are prepared by fusing spleen cells from the immunized animal with myeloma cells. The fused products are screened for those producing antibodies that bind to the immunogen. The positive hybridoma clones are isolated, and the monoclonal antibodies are recovered from those clones.

Immunization regimens for production of both polyclonal and monoclonal antibodies are well-known in the art. The immunogen may be injected by any of a number of routes, including subcutaneous, intravenous, intraperitoneal, intradermal, intramuscular, mucosal, or a combination thereof. The immunogen may be injected in soluble form, aggregate form, attached to a physical carrier, or mixed with an adjuvant, using methods and materials well-known in the art. The antisera and antibodies may be purified using column chromatography methods well known to those skilled in the art.

According to the present invention, antibody fragments which contain specific binding sites for the polypeptides or fragments thereof may also be generated. For example, such fragments can be F(ab')₂ fragments produced by pepsin digestion of the antibody molecule or Fab fragments generated by reducing the disulfide bridges of the F(ab')₂ fragments.

The subject invention also provides methods for diagnosing the diseases associated with Hippocalcin-V, for example the large cell lung carcinoma, small cell lung carcinoma, colon adenocarcinoma and promonocytic leukemia, by utilizing the nucleic acid sequences, the polypeptide of the human Hippocalcin-V, or fragments thereof, and the antibodies against the polypeptides.

Since Hippocalcin-V clone was isolated from a small cell lung carcinoma cell line (H209), it is advisable that Hippocalcin-V may serve as a marker for the diagnosis of human small cell lung carcinoma. Thus, the expression level of Hippocalcin-V relative to Hippocalcin may be a useful indicator for screening patients suspected of having cancers such as large cell lung carcinoma, small cell lung carcinoma, colon adenocarcinoma and promonocytic leukemia. This suggests that the index of relative expression level (mRNA or protein) may confer an increased susceptibility to large cell lung carcinoma, small cell lung carcinoma, colon adenocarcinoma and promonocytic leukemia. Fragments of Hippocalcin-V transcripts (mRNAs) may be detected by RT-PCR approach. Polypeptides of Hippocalcin-V may be determined by the binding of antibodies to these polypeptides. Furthermore, a method (RNAi; RNA interference) that is known to those skilled in the art may be used to interfere and degrade the targeted RNA using chemically synthesized double stranded short nucleic acid (about 23 nucleotides dsRNA) molecules. According to the present invention, the double stranded short nucleic acid molecules contain the sequence of the "gene target": nucleotides 18-19 of Hippocalcin-V, may be used to specifically interfere and degrade Hippocalcin-V mRNA. After the RNAi approach is conducted, the decreased transcripts or polypeptides may be used to interpret the presence of Hippocalcin-V mRNA.

According to the present invention, the expression of these gene variant mRNAs may be determined by RT-PCR. Using TRIZOL reagents (Life Technology), total RNA may be isolated from patient samples. Tissue samples (e.g., biopsy samples) are powdered under liquid nitrogen before homogenization. RNA purity and integrity are assessed by absorbance at 260/280 nm and by agarose gel electrophoresis. A set of primers can be designed to amplify the expected size of specific PCR fragments of Hippocalcin-V. For example, one of the primers is a fragment of the sequence (A fragment; forward primer) containing the gene target: nucleotides 18-19 of Hippocalcin-V, and the other is a fragment (B fragment; reverse primer) complementary to the Hippocalcin-V sequence designed at any location downstream of "A fragment". Alternatively, one primer (A fragment; forward primer) is designed at any location upstream of the sequence containing the gene target and the other is designed at any location complementary to a portion downstream of the sequence containing the gene target. The length of the PCR fragment from Hippocalcin-V will be 399bp shorter than that from Hippocalcin. PCR fragments are analyzed on a 1 % agarose gel using five microliters (10%) of the amplified products. The intensity of the signals may be determined by using the Molecular Analyst program (version 1.4.1; Bio-Rad). Thus, the index of relative expression levels for each co-amplified PCR products may be calculated based on the signal intensities.

The RT-PCR experiment may be performed according to the manufacturer instructions (Boehringer Mannheim). A 50µl reaction mixture containing 2µl total RNA (0.1 µl/µl), 1 µl each primer (20 pM), 1 µl each dNTP (10 mM), 2.5 µl DTT solution (100 mM), 10 µl 5X RT-PCR buffer, 1µl enzyme mixture, and 28.5 µl sterile distilled water may be subjected to the conditions such as reverse transcription at 60°C for 30 minutes followed by 35 cycles of denaturation at 94°C for 2 minutes, annealing at 60°C for 2 minutes, and extension at 68°C for 2 minutes. The RT-PCR analysis may be repeated twice to ensure reproducibility, for a total of three independent experiments.

The expression of gene variants can also be analyzed using Northern Blot hybridization approach. Specific fragment of the Hippocalcin-V may be amplified by polymerase chain reaction (PCR) using primer set designed for RT-PCR. The amplified PCR fragment may be labeled and served as a probe to hybridize the membranes containing total RNAs extracted from the samples under the conditions of 55°C in a suitable hybridization solution for 3 hours. Blots may be washed twice in 2 x SSC, 0.1% SDS at room temperature for 15 minutes each, followed by two washes in 0.1 x SSC and 0.1% SDS at 65°C for 20 minutes each. After these washes, blot may be rinsed briefly in suitable washing buffer and incubated in blocking solution for 30 minutes. Then it is incubated in suitable antibody solution for 30 minutes. Blots may be washed in washing buffer for 30 minutes and equilibrated in suitable detection buffer before detecting the signals. Alternatively, the presence of gene variants (cDNAs or PCR) can be detected using microarray approach. The cDNAs or PCR products corresponding to the nucleotide sequences of the present invention may be immobilized on a suitable substrate such as a glass slide. Hybridization can be preformed using the labeled mRNAs extracted from samples. After hybridization, nonhybridized mRNAs are removed. The relative abundance of each labeled transcript, hybridizing to a cDNA/PCR product immobilized on the microarray, can be determined by analyzing the scanned images.

According to the present invention, the presence of the polypeptide of Hippocalcin-V in samples may be determined by, but not limited to, the immunoassay which uses the antibody that specifically binds to the polypeptide. The polypeptides of the gene variants may be expressed in prokaryotic cells by using suitable prokaryotic expression vectors. The cDNA fragments of Hippocalcin-V gene encoding the amino acid coding sequence may be PCR amplified using primer set A with restriction enzyme digestion sites incorporated in the 5' and 3' ends. The PCR products can then be enzyme digested, purified, and inserted into the corresponding sites of prokaryotic expression vector in-frame to generate recombinant plasmids. Sequence fidelity of this recombinant DNA can be verified by sequencing. The prokaryotic recombinant plasmids may be transformed into host cells (e.g., *E*. *coli* BL21 (DE3)). Recombinant protein synthesis may be stimulated by the addition of 0.4 mM isopropylthiogalactoside (IPTG) for 3 hours. The bacterially-expressed proteins may be purified.

The polypeptide of the gene variant may be expressed in animal cells by using eukaryotic expression vectors. Cells may be maintained in Dulbecco's modified Eagle's medium (DMEM) supplemented with 10% fetal bovine serum (FBS; Gibco BRL) at 37°C in a humidified 5% CO₂ atmosphere. Before transfection, the nucleotide sequence of each of the gene variant may be amplified with PCR primers containing restriction enzyme digestion sites and ligated into the corresponding sites of eukaryotic expression vector in-frame. Sequence fidelity of this recombinant DNA can be verified by sequencing. The cells may be plated in 12-well plates one day before transfection at a density of 5 ×10⁴ cells per well. Transfections may be carried out using Lipofectaminutese Plus transfection reagent according to the manufacturer's instructions (Gibco BRL). Three hours following transfection, the medium containing the complexes may be replaced with fresh medium. Forty-eight hours after incubation, the cells may be scraped into lysis buffer (0.1 M Tris HCl, pH 8.0, 0.1% Triton X-100) for purification of expressed proteins. After these proteins are purified, monoclonal antibodies against these purified proteins (Hippocalcin-V) may be generated using hybridoma technique according to the conventional methods.

According to the present invention, the presence of the polypeptides of Hippocalcin-V in samples of the large cell lung carcinoma, small cell lung carcinoma, colon adenocarcinoma and promonocytic leukemia may be determined by a Western blot analysis. Proteins extracted from these samples may be separated by SDS-PAGE and transferred to suitable membranes such as polyvinylidene difluoride (PVDF) in transfer buffer (25 mM Tris-HCl, pH 8.3, 192 mM glycine, 20% methanol) with a Trans-Blot apparatus for 1 hour at 100 V (e.g., Bio-Rad). The proteins can be immunoblotted with specific antibodies. For example, membrane blotted with extracted proteins may be blocked with suitable buffers such as 3% solution of BSA or 3% solution of nonfat milk powder in TBST buffer (10 mM Tris-HCl, pH 8.0, 150 mM NaCl, 0.1 % Tween 20) and incubated with monoclonal antibody directed against the polypeptides of gene variants. Unbound antibody is removed by washing with TBST for 5 X 1 minutes. Bound antibody may be detected using commercial ECL Western blotting detecting reagents.

The following examples are provided for illustration, but not for limiting the invention.

### EXAMPLES

### Analysis of Human Small Cell Lung Carcinoma EST Database

Expressed sequence tags (ESTs) generated from the large-scale PCR-based sequencing of the 5'-end of human small cell lung carcinoma cDNA clones were compiled and served as EST databases. Sequence comparisons against the nonredundant nucleotide and protein databases were performed using BLASTN and BLASTX programs, at the National Center for Biotechnology Information (NCBI) with a significance cutoff of p<10⁻¹⁰. ESTs representing putative Hippocalcin-V gene were identified during the course of EST generation.

### Isolation of cDNA Clones

One cDNA clone exhibiting EST sequence similar to the Hippocalcin gene was isolated from the human small cell lung carcinoma cDNA library and named Hippocalcin-V. The inserts of these clones were subsequently excised *in vivo* from the λZAP Express vector using the ExAssist/XLOLR helper phage system (Stratagene). Phagemid particles were excised by coinfecting XL1-BLUE MRF' cells with ExAssist helper phage. The excised pBluescript phagemids were used to infect *E*. *coli* XLOLR cells, which lack the amber suppressor necessary for ExAssist phage replication. Infected XLOLR cells were selected using kanamycin resistance. Resultant colonies contained the double stranded phagemid vector with the cloned cDNA insert. A single colony was grown overnight in LB-kanamycin, and DNA was purified using a Qiagen plasmid purification kit.

### Full Length Nucleotide Sequencing and Database Comparisons

Phagemid DNA was sequenced using the *Taq* dye-deoxy terminator cycle sequencing kit for the Applied Biosystems 377 sequencing system (PerkinElmer Life Sciences). Using the primer-walking approach, full-length sequence was determined. Nucleotide and protein searches were performed using BLAST against the non-redundant database of NCBI.

### In Silico Sequence Similarity Analysis

The coding sequence for each cDNA clones was searched against the dbEST sequence database using the BLAST algorithm at the NCBI website. ESTs derived from dbEST sequence database were used as a source of information for transcript sequence similarity analysis. The accession number of the ESTs matching to the deleted sequence of Hippocalcin-V was identified.

### RT-PCR Expression Pattern Analysis

The expression pattern of Hippocalcin-V was conducted in human cell lines using RT-PCR. The human cell lines were WI38 (Lung, fetus); A549 (Lung adenocarcinoma); H661 (Large cell carcinoma, lung); H520 (Squamous cell carcinoma, lung); H209 (Small cell carcinoma, lung); JHH-4 (Hepatoma); SUP-T1 (T-cell lymophoblastic lymphoma); Daudi (Burkitt's lymophoma); Ramos (Burkitt's lymophoma); RAJI (Burkitt's lymophoma); ZR75-1 (Breast carcinoma); MDA-MB-231 (Breast adenocarcinoma); Hs 578T (Breast carcinoma); G5T/VGH (Glioblastoma multiforme); TSGH9201 (Gastric carcinoma); Ca Ski (Cervix epidermoid carcinoma); Hela S3 (Cervical epitheloid carcinoma); COLO 320 HSR (Colon adenocarcinoma); SW620 (Colon adenocarcinoma); TSGH8301 (Urinary bladder carcinoma); ES-2 (Ovarian carcinoma); DU145 (Brain prostate carcinoma); MIA paca-2 (Pancreatic carcinoma); CE48T/VGH (Esophagus epidermoid carcinoma); CE81T/VGH (Esophagus carcinoma well differentiated aquamous); HL-CZ (Promonocytic leukemia); Hs 181.tes (Normal testis). The purity and integrity of total RNA extracted from each of the cell lines were assessed by absorbance at 260/280 nm and by agarose gel electrophoresis. The forward and reverse primers for Hippocalcin-V were: 5'-GGTGTCTCCGCGTCGCTG-3' and 5 '-GCCCCGCGGATGAACTCC-3'. The expected size of Hippocalcin-V PCR fragments was 159bp.

Glyceraldehyde-3-phosphate dehydrogenase (GAPDH; accession No. M33197) was used for internal control. The forward and reverse primers for GAPDH were: 5'-TGGGTGTGAACCATGAGAAG-3' and 5'-GTGTCGCTGTTGAAGTCAGA-3'. The expected size of GAPDH PCR fragment was 472 bp. Briefly, a 50 µl reaction mixture containing 2µl total RNA (0.1µg/µl), 1µl each primer (20 pM), 1µl each dNTP (10 mM), 2.5 µl DTT solution (100 mM), 10 µl 5X RT-PCR buffer, 1µl enzyme mixture and 28.5 µl sterile redistilled water were subjected to reverse transcription at 60°C for 30 minutes followed by 35 cycles of denaturation at 94°C for 2 minutes, annealing at 60°C for 2 minutes, and extension at 68°C for 2 minutes. Five microliters (10%) of the amplified products mixed with 1 µl of loading buffer were separated on a 1 % horizontal agarose gel stained with ethidium bromide in 0.5X TAE buffer. The gel was electrophoresed at 100 V for 45 minutes.

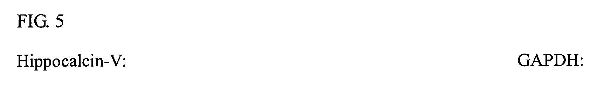

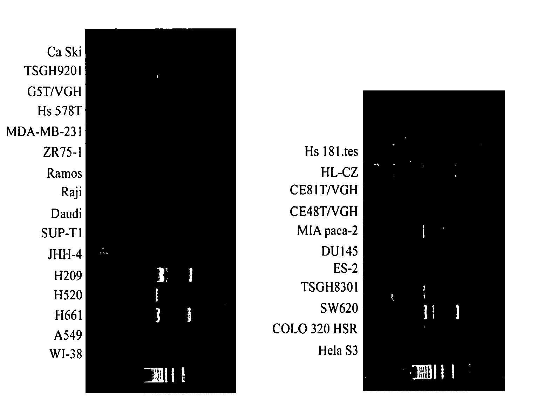

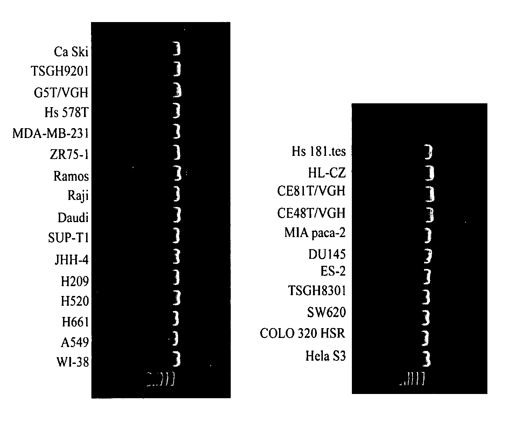

Figure 5 showed a coamplification of both Hippocalcin (558bp) and Hippocalcin-V (159bp) mRNAs (shown on the left are 100 bp DNA ladder markers). The fragments of 558bp and 159bp for Hippocalcin and Hippocalcin-V, respectively, were confirmed by sequencing. Hippocalcin mRNA was expressed in the cell lines of the large cell lung carcinoma, squamous cell lung carcinoma, small cell lung carcinoma, gastric carcinoma, colon adenocarcinoma (COLO-320-HSR; SW620), urinary bladder carcinoma, pancreatic carcinoma and promonocytic leukemia. It is interesting to note that the Hippocalcin-V mRNA was only expressed in the cell lines of the large cell lung carcinoma, small cell lung carcinoma, colon adenocarcinoma (SW620) and promonocytic leukemia. The different expression patterns between Hippocalcin-V and Hippocalcin implied that they may be functionally different. The expression pattern of Hippocalcin-V suggests that Hippocalcin-V may be a suitable marker for diagnosing human large cell lung carcinoma, small cell lung carcinoma, colon adenocarcinoma and promonocytic leukemia.

### REFERENCES

Burgoyne and Weiss, The neuronal calcium sensor family of Ca2+-binding proteins. Biochem J. 353:1-12, (2001).
Maeda et al. Aberrant expression of photoreceptor-specific calcium-binding protein (recoverin) in cancer cell lines. Cancer Res. 60:1914-20, (2000).
Maeda et al. Mechanisms of Photoreceptor Cell Death in Cancer-Associated Retinopathy. Investigative Ophthalmology and Visual Science.;42:705-712, (2001).
Mercer et al., NAIP interacts with hippocalcin and protects neurons against calcium-induced cell death through caspase-3-dependent and -independent pathways. EMBO J. 19:3597-607, (2000).
Ohguro et al. Clinicopathological features of gastric cancer cases and aberrantly expressed recoverin. Tohoku J Exp Med. 202:213-9, (2004).
Polans et al., Recoverin, a photoreceptor-specific calcium-binding protein, is expressed by the tumor of a patient with cancer-associated retinopathy. Proc Natl Acad Sci USA. 92:9176-80, (1995).

## Claims

1. An isolated polypeptide comprising the amino acid sequence of SEQ ID NO: 4.

2. An isolated nucleic acid encoding said polypeptide of Claim 1.

3. The isolated nucleic acid of Claim 2, comprising the nucleotide sequence of SEQ ID NO: 3.

4. An isolated nucleic acid comprising at least 30 consecutive nucleotide and that includes a di-nucleotide of nucleotides 18 to 19 of SEQ ID NO: 3.

5. An isolated polypeptide comprising the polypeptide encoded by said nucleic acid of claim 4.

6. A purified polypeptide comprising the polypeptide encoded by said nucleic acid of claim 4.

7. An expression vector comprising the nucleic acid of any one of claims 2 to 4.

8. A host cell comprising said expression vector of Claim 7.

9. A method for producing a Hippocalcin variant peptide, comprising the steps of:
(1) culturing the host cell of Claim 8 under a condition suitable for the expression of a Hippoclacin variant peptide; and
(2) recovering said Hippocalcin variant peptide from the host cell culture.

10. An antibody that specifically binds to said polypeptide of Claim 6.

11. The antibody of Claim 10, which is a polyclonal or monoclonal antibody.

12. A method for detecting the presence of a Hippocalcin nucleic acid in a mammal, comprising the steps of:
(a) extracting total RNA from a sample obtained from said mammal;
(b) amplifying said RNA by reverse transcriptase-polymerase chain reaction (RT-PCR) to obtain a cDNA sample;
(c) hybridizing said cDNA sample with the nucleic acid of claim 4; and (4) detecting said hybridization.

13. The method of Claim 12, wherein said hybridizing process is conducted by Northern blot or microarray to diagnose a plurality of cancers.

14. The method of Claim 13, wherein said plurality of cancers include large cell lung carcinoma, small cell lung carcinoma, colon adenocarcinoma and promonocytic leukemia.

15. A method for detecting the presence of a Hippocalcin polypeptide or fragments thereof in a mammal, which comprises the steps of:
(a) extracting proteins from a mammal;
(b) contacting said proteins with an antibody that binds to a Hippocalcin antigen comprising a polypeptide having at least 17 contiguous and unique amino acids from SEQ ID NO: 4 that are not found in SEQ ID NO:2; and
(c) detecting said antibody-Hippocalcin antigen binding.

16. The method of Claim 15, wherein said antibody-antigen binding samples are detected by Western blot approach to help diagnose a plurality of cancers.

17. The method of Claim 16, wherein said plurality of cancers include large cell lung carcinoma, small cell lung carcinoma, colon adenocarcinoma and promonocytic leukemia.


SEQUENCE LISTING

<110> VISGENEER, INC

<120> HUMAN HIPPOCALCIN-RELATED GENE VARIANT ASSOCIATED WITH CANCERS

<130> none

<140> EP 06018330.8-2405
<141> 2006-09-01

<160> 8

<170> PatentIn version 3.3

<210> 1
<211> 1440
<212> DNA
<213> Homo sapiens

<400> 1
ggtgtctccg cgtcgctggg tgggacttgg ctcggcggcc atgggcaagc agaacagcaa 60

gctgcggccc gagatgttgc aggacctgcg agagaacaca gagttctcag agctggagct 120

gcaggagtgg tacaagggct tcctcaagga ctgccccaca ggaatcctca atgtggatga 180

gttcaagaag atctacgcca acttctttcc ctatggtgac gcctccaagt ttgccgagca 240

cgtcttccgc acctttgaca ccaacagcga tggcaccata gactttcggg agttcatcat 300

tgcgctgagc gtgacctcgc gcggccgcct ggagcagaag ctcatgtggg ccttcagcat 360

gtatgacctg gacggcaacg gctacatcag ccgggaggag atgctggaga tcgtgcaggc 420

catttacaag atggtttcgt ccgtgatgaa gatgccggag gacgagtcga ccccggaaaa 480

gaggactgag aaaatcttcc gccaaatgga cacaaacaac gacggcaagc tgtccttgga 540

ggagttcatc cgcggggcca aaagcgaccc gtccatcgtg cgtctgctgc agtgcgaccc 600

cagcagcgcc tcccagttct gagaggagcc aggttcccct tcctccctcc cttcaccggc 660

cccctcccgg ctcttagctt ccactccctt gtgtgtattc tggctggggg ccagattggg 720

gaagcccttc tccccgggtc tgccctgtgg ggggcttccg gaaaagggaa ccctgcggta 780

cccccaggcc aaagcaagta agcggttagc accccccaat cccagaggca acaatagaga 840

cacaggctgg gttggtctgc ccctcagtca ggccccctta cccacccacc agccccaagg 900

cccgacccct cccccaaagg ggcagtcccc ttcttgcagg tctcagcttg cggggtgggg 960

ggagtcatgc ccaggggagg agacttttta tctggagggg agagaaggat tctaggggtg 1020

tggagttgga gaaagaggct tccttgagcc acccttccca ccccagccct tgctggtccc 1080

taggccaagc caccaagtga aaccttccag gatactagcc cgccagctgt gggccccaga 1140

aagccagcct gccttttagc acttggatac acacagaccc acggagctct ctgtgtttgg 1200

cctctcacac acacacaggc ccatagacag atgggcagat gcacccagag ccccatgcac 1260

agtgtcctgc gtgggaaaga gacacggtcc ccctggctga gcccctgtcc tcccctctgt 1320

ccccccaacc gccccccctg catgcagcca aatggagcat ctctgttctt tttaataatt 1380

tcagaataaa gtctcatttc agtgcaaaaa aaaaaaaaaa aaaaaaaaaa aaaaaaaaaa 1440

<210>  2
<211>  193
<212>  PRT
<213>  Homo sapiens

<400>  2

Met Gly Lys Gln Asn Ser Lys Leu Arg Pro Glu Met Leu Gln Asp Leu
1               5                   10                  15

Arg Glu Asn Thr Glu Phe Ser Glu Leu Glu Leu Gln Glu Trp Tyr Lys
            20                  25                  30

Gly Phe Leu Lys Asp Cys Pro Thr Gly Ile Leu Asn Val Asp Glu Phe
        35                  40                  45

Lys Lys Ile Tyr Ala Asn Phe Phe Pro Tyr Gly Asp Ala Ser Lys Phe
    50                  55                  60

Ala Glu His Val Phe Arg Thr Phe Asp Thr Asn Ser Asp Gly Thr Ile
65                  70                  75                  80

Asp Phe Arg Glu Phe Ile Ile Ala Leu Ser Val Thr Ser Arg Gly Arg
                85                  90                  95

Leu Glu Gln Lys Leu Met Trp Ala Phe Ser Met Tyr Asp Leu Asp Gly
            100                 105                 110

Asn Gly Tyr Ile Ser Arg Glu Glu Met Leu Glu Ile Val Gln Ala Ile
        115                 120                 125

Tyr Lys Met Val Ser Ser Val Met Lys Met Pro Glu Asp Glu Ser Thr
    130                 135                 140

Pro Glu Lys Arg Thr Glu Lys Ile Phe Arg Gln Met Asp Thr Asn Asn
145                 150                 155                 160

Asp Gly Lys Leu Ser Leu Glu Glu Phe Ile Arg Gly Ala Lys Ser Asp
                165                 170                 175

Pro Ser Ile Val Arg Leu Leu Gln Cys Asp Pro Ser Ser Ala Ser Gln
            180                 185                 190

Phe

<210>  3
<211>  1041
<212>  DNA
<213>  Homo sapiens

<400>  3
ggtgtctccg cgtcgctggg ccatttacaa gatggtttcg tccgtgatga agatgccgga      60

ggacgagtcg accccggaaa agaggactga gaaaatcttc cgccaaatgg acacaaacaa 120

cgacggcaag ctgtccttgg aggagttcat ccgcggggcc aaaagcgacc cgtccatcgt 180

gcgtctgctg cagtgcgacc ccagcagcgc ctcccagttc tgagaggagc caggttcccc 240

ttcctccctc ccttcaccgg ccccctcccg gctcttagct tccactccct tgtgtgtatt 300

ctggctgggg gccagattgg ggaagccctt ctccccgggt ctgccctgtg gggggcttcc 360

ggaaaaggga accctgcggt acccccaggc caaagcaagt aagcggttag caccccccaa 420

tcccagaggc aacaatagag acacaggctg ggttggtctg cccctcagtc aggccccctt 480

acccacccac cagccccaag gcccgacccc tcccccaaag gggcagtccc cttcttgcag 540

gtctcagctt gcggggtggg gggagtcatg cccaggggag gagacttttt atctggaggg 600

gagagaagga ttctaggggt gtggagttgg agaaagaggc ttccttgagc cacccttccc 660

accccagccc ttgctggtcc ctaggccaag ccaccaagtg aaaccttcca ggatactagc 720

ccgccagctg tgggccccag aaagccagcc tgccttttag cacttggata cacacagacc 780

cacggagctc tctgtgtttg gcctctcaca cacacacagg cccatagaca gatgggcaga 840

tgcacccaga gccccatgca cagtgtcctg cgtgggaaag agacacggtc cccctggctg 900

agcccctgtc ctcccctctg tccccccaac cgccccccct gcatgcagcc aaatggagca 960

tctctgttct ttttaataat ttcagaataa agtctcattt cagtgcaaaa aaaaaaaaaa 1020

aaaaaaaaaa aaaaaaaaaa a 1041

<210> 4
<211> 63
<212> PRT
<213> Homo sapiens

<400> 4

Met Val Ser Ser Val Met Lys Met Pro Glu Asp Glu Ser Thr Pro Glu
1               5                   10                  15

Lys Arg Thr Glu Lys Ile Phe Arg Gln Met Asp Thr Asn Asn Asp Gly
            20                  25                  30

Lys Leu Ser Leu Glu Glu Phe Ile Arg Gly Ala Lys Ser Asp Pro Ser
        35                  40                  45

Ile Val Arg Leu Leu Gln Cys Asp Pro Ser Ser Ala Ser Gln Phe
    50                  55                  60

<210> 5
<211> 18
<212> DNA
<213> Homo sapiens

<400> 5
ggtgtctccg cgtcgctg 18

<210>  6
<211>  18
<212>  DNA
<213>  Homo sapiens

<400>  6
gccccgcgga tgaactcc                                                    18


<210>  7
<211>  20
<212>  DNA
<213>  Homo sapiens

<400>  7
tgggtgtgaa ccatgagaag                                                  20


<210>  8
<211>  20
<212>  DNA
<213>  Homo sapiens

<400>  8
gtgtcgctgt tgaagtcaga                                                  20

SEQUENCE LISTING

<110>  VISGENEER, INC

<120>  HUMAN HIPPOCALCIN-RELATED GENE VARIANT ASSOCIATED WITH CANCERS

<130>  none

<160>  4

<170>  PatentIn version 3.3

<210>  1
<211>  1440
<212>  DNA
<213>  Homo sapiens

<400>  1
ggtgtctccg cgtcgctggg tgggacttgg ctcggcggcc atgggcaagc agaacagcaa      60

gctgcggccc gagatgttgc aggacctgcg agagaacaca gagttctcag agctggagct     120

gcaggagtgg tacaagggct tcctcaagga ctgccccaca ggaatcctca atgtggatga     180

gttcaagaag atctacgcca acttctttcc ctatggtgac gcctccaagt ttgccgagca     240

cgtcttccgc acctttgaca ccaacagcga tggcaccata gactttcggg agttcatcat     300

tgcgctgagc gtgacctcgc gcggccgcct ggagcagaag ctcatgtggg ccttcagcat     360

gtatgacctg gacggcaacg gctacatcag ccgggaggag atgctggaga tcgtgcaggc     420

catttacaag atggtttcgt ccgtgatgaa gatgccggag gacgagtcga ccccggaaaa     480

gaggactgag aaaatcttcc gccaaatgga cacaaacaac gacggcaagc tgtccttgga     540

ggagttcatc cgcggggcca aaagcgaccc gtccatcgtg cgtctgctgc agtgcgaccc     600

cagcagcgcc tcccagttct gagaggagcc aggttcccct tcctccctcc cttcaccggc     660

cccctcccgg ctcttagctt ccactccctt gtgtgtattc tggctggggg ccagattggg     720

gaagcccttc tccccgggtc tgccctgtgg ggggcttccg gaaaagggaa ccctgcggta     780

cccccaggcc aaagcaagta agcggttagc accccccaat cccagaggca acaatagaga     840

cacaggctgg gttggtctgc ccctcagtca ggccccctta cccacccacc agccccaagg     900

cccgacccct cccccaaagg ggcagtcccc ttcttgcagg tctcagcttg cggggtgggg     960

ggagtcatgc ccaggggagg agacttttta tctggagggg agagaaggat tctaggggtg    1020

tggagttgga gaaagaggct tccttgagcc acccttccca ccccagccct tgctggtccc    1080

taggccaagc caccaagtga aaccttccag gatactagcc cgccagctgt gggccccaga    1140

aagccagcct gccttttagc acttggatac acacagaccc acggagctct ctgtgtttgg    1200

cctctcacac acacacaggc ccatagacag atgggcagat gcacccagag ccccatgcac    1260

agtgtcctgc gtgggaaaga gacacggtcc ccctggctga gcccctgtcc tcccctctgt    1320

ccccccaacc gccccccctg catgcagcca aatggagcat ctctgttctt tttaataatt    1380

tcagaataaa gtctcatttc agtgcaaaaa aaaaaaaaaa aaaaaaaaaa aaaaaaaaaa    1440

<210>  2

<211>  193
<212>  PRT
<213>  Homo sapiens

<400>  2

Met Gly Lys Gln Asn Ser Lys Leu Arg Pro Glu Met Leu Gln Asp Leu
1               5                   10                  15

Arg Glu Asn Thr Glu Phe Ser Glu Leu Glu Leu Gln Glu Trp Tyr Lys
            20                  25                  30

Gly Phe Leu Lys Asp Cys Pro Thr Gly Ile Leu Asn Val Asp Glu Phe
        35                  40                  45

Lys Lys Ile Tyr Ala Asn Phe Phe Pro Tyr Gly Asp Ala Ser Lys Phe
    50                  55                  60

Ala Glu His Val Phe Arg Thr Phe Asp Thr Asn Ser Asp Gly Thr Ile
65                  70                  75                  80

Asp Phe Arg Glu Phe Ile Ile Ala Leu Ser Val Thr Ser Arg Gly Arg
                85                  90                  95

Leu Glu Gln Lys Leu Met Trp Ala Phe Ser Met Tyr Asp Leu Asp Gly
            100                 105                 110

Asn Gly Tyr Ile Ser Arg Glu Glu Met Leu Glu Ile Val Gln Ala Ile
        115                 120                 125

Tyr Lys Met Val Ser Ser Val Met Lys Met Pro Glu Asp Glu Ser Thr
    130                 135                 140

Pro Glu Lys Arg Thr Glu Lys Ile Phe Arg Gln Met Asp Thr Asn Asn
145                 150                 155                 160

Asp Gly Lys Leu Ser Leu Glu Glu Phe Ile Arg Gly Ala Lys Ser Asp
                165                 170                 175

Pro Ser Ile Val Arg Leu Leu Gln Cys Asp Pro Ser Ser Ala Ser Gln
            180                 185                 190

Phe


<210>  3
<211>  1041
<212>  DNA
<213>  Homo sapiens

<400>  3
ggtgtctccg cgtcgctggg ccatttacaa gatggtttcg tccgtgatga agatgccgga      60

ggacgagtcg accccggaaa agaggactga gaaaatcttc cgccaaatgg acacaaacaa     120

cgacggcaag ctgtccttgg aggagttcat ccgcggggcc aaaagcgacc cgtccatcgt 180

gcgtctgctg cagtgcgacc ccagcagcgc ctcccagttc tgagaggagc caggttcccc 240

ttcctccctc ccttcaccgg ccccctcccg gctcttagct tccactccct tgtgtgtatt 300

ctggctgggg gccagattgg ggaagccctt ctccccgggt ctgccctgtg gggggcttcc 360

ggaaaaggga accctgcggt acccccaggc caaagcaagt aagcggttag caccccccaa 420

tcccagaggc aacaatagag acacaggctg ggttggtctg cccctcagtc aggccccctt 480

acccacccac cagccccaag gcccgacccc tcccccaaag gggcagtccc cttcttgcag 540

gtctcagctt gcggggtggg gggagtcatg cccaggggag gagacttttt atctggaggg 600

gagagaagga ttctaggggt gtggagttgg agaaagaggc ttccttgagc cacccttccc 660

accccagccc ttgctggtcc ctaggccaag ccaccaagtg aaaccttcca ggatactagc 720

ccgccagctg tgggccccag aaagccagcc tgccttttag cacttggata cacacagacc 780

cacggagctc tctgtgtttg gcctctcaca cacacacagg cccatagaca gatgggcaga 840

tgcacccaga gccccatgca cagtgtcctg cgtgggaaag agacacggtc cccctggctg 900

agcccctgtc ctcccctctg tccccccaac cgccccccct gcatgcagcc aaatggagca 960

tctctgttct ttttaataat ttcagaataa agtctcattt cagtgcaaaa aaaaaaaaaa 1020

aaaaaaaaaa aaaaaaaaaa a 1041

<210> 4
<211> 63
<212> PRT
<213> Homo sapiens

<400> 4

Met Val Ser Ser Val Met Lys Met Pro Glu Asp Glu Ser Thr Pro Glu
1               5                   10                  15

Lys Arg Thr Glu Lys Ile Phe Arg Gln Met Asp Thr Asn Asn Asp Gly
            20                  25                  30

Lys Leu Ser Leu Glu Glu Phe Ile Arg Gly Ala Lys Ser Asp Pro Ser
        35                  40                  45

Ile Val Arg Leu Leu Gln Cys Asp Pro Ser Ser Ala Ser Gln Phe
    50                  55                  60